# EUROPEAN PATENT APPLICATION

(11) **EP 3 287 138 A1**
(43) Date of publication of application: **28.02.2018**
(21) Application number: 17194652.8
(22) Date of filing: 22.08.2013
(51) Int. Cl.: A61K 38/15, A61K 31/355, A61K 9/19

(54) **IMPROVED DAPTOMYCIN INJECTABLE FORMULATION**

(30) Priority: 23.08.2012 IN 2452MU2012
(62) Divisional of application: 13838871.5
(71) Applicant: Agila Specialties Private Limited, Karnataka 560 076 (IN)
(72) Inventor: CHETLAPALLI, Satya Srinivas, Bangladore, Karnataka 560 076 (IN); MANDAVILLI, Srirama Sarveswara Rao, Bangladore, Karnataka 560 076 (IN); JUSTIN, Babu, Bangladore, Karnataka 560 076 (IN); MEDA, Sathyanarayan Srinivas, Bangladore, Karnataka 560 076 (IN)
(74) Representative: Cooke, Richard Spencer

(57) **Abstract**

The present invention discloses a lyophilized pharmaceutical formulation comprising antibacterial agent, daptomycin as active and tocopheryl phosphate hydrolysate mixture with improved reconstitution time for parental administration and to the process of preparation thereof.

## Description

### FIELD OF THE INVENTION:

The present invention relates to a lyophilized pharmaceutical formulation comprising antibacterial agent, daptomycin and tocopheryl phosphate hydrolysate mixture (TPM). More particularly, the invention relates to stable, lyophilized daptomycin formulation with improved reconstitution time and to the process of preparation thereof.

### BACKGROUND AND PRIOR ART:

Daptomycin is a cyclic lipopeptide antibacterial agent derived from the fermentation of Streptomyces roseosporus. The chemical name is N-decanoyl-L-tryptophyl-D-asparaginyl-L-aspartyl-L-threonylglycyl-L-ornithyl-L-aspartyl-D-alanyl-L-aspartylglycyl-D-seryl-threo-3-methyl -L-glutamyl-3-anthraniloyl-L-alanine ε1-lactone. The chemical structure is:

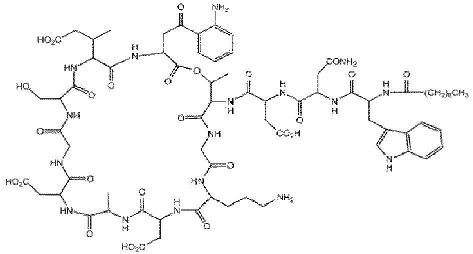

Daptomycin is first disclosed in US4537717. The empirical formula is C₇₂H₁₀₁N₁₇O₂₆; the molecular weight is 1620.67

Daptomycin is marketed in the United States under the trade name Cubicin in the form of injection containing 500mg/vial marketed by Cubist Pharmaceuticals Inc. In Europe, Cubicin is marketed by Novartis Europharm as Cubicin powder that is made up into a solution for injection or infusion (drip into a vein). The single vials contain 350mg or 500mg of the active, Daptomycin. In Australia, cubicin is marketed by Novartis Pharmaceuticals, Australia Pty limited as injectables containing 350mg or 500mg of active, daptomycin per vial.
The currently marketed formulations of injectable Daptomycin are supplied in a single-use vial as a sterile, preservative-free, pale yellow to light brown, lyophilized cake containing approximately 500 mg or 350mg of daptomycin for intravenous (IV) use following reconstitution with 0.9% sodium chloride injection. The only inactive ingredient is sodium hydroxide, which is used in minimal quantities for pH adjustment. Freshly reconstituted solutions of Cubicin range in color from pale yellow to light brown.

During the preparation of Cubicin for administration, it is essential that the contents of the vial should be reconstituted using aseptic technique which is described below:
i. Avoiding vigorous agitation or shaking of the vial during or after reconstitution to minimize foaming;
ii. Removing the polypropylene flip-off cap from the vial to expose the central portion of the rubber stopper;
iii. Slowly transferring 10 mL of 0.9% sodium chloride injection through the center of the rubber stopper into the vial, pointing the transfer needle towards the wall of the vial;
iv. Ensuring that the entire product is wetted by gently rotating the vial;
v. Allowing the product to stand undisturbed for 10 minutes; and
vi. Gently rotating or swirling the vial contents for a few minutes, as needed, to obtain a completely reconstituted solution.

The aseptic technique mentioned above for the preparation of final intravenous (IV) solution is tedious. Further, as per Cubicin's pack insert the reconstituted solution is stable in the vial for 12 hours at room temperature and up to 48 hours if stored under refrigeration at 2 to 8°C (36 to 46°F).

Thus as indicated above, the major drawbacks of the innovator formulation is the long reconstitution procedure where the finished product to be administered needs to be reconstituted which takes about 10 min followed by gentle rotation or swirling of the vial contents for another few minutes, as needed, to obtain a completely clear solution for administration. Thus too much time is wasted before the product is administered to the patient who could be critical during emergency cases as the drug is indicated for treating Staphylococcus aureus bloodstream infections and complicated skin and skin structure infections (cSSSI). Also, it increases the product exposure to room temperature which could lead to product degradation.

In view of the above, it is the object of the present invention to provide a daptomycin parental formulation that can be directly reconstituted with a vehicle with a reduced reconstitution time.

The other object of the invention is to increase the stability of the product at room temperature and to increase the rate of solubilization of lyophilized product thereby reducing the time required for reconstitution.

### SUMMARY OF THE INVENTION:

In accordance with the objective, the present invention provides a lyophilized parental formulation consisting of antibacterial agent daptomycin as active, tocopheryl phosphate hydrolysate mixture (TPM), which acts as an antioxidant as well as solubilizer. The present composition increases the stability of the product at room temperature after reconstitution, improves the reconstitution time, thus accelerating the administration process to the patient in need of

In another aspect, the present invention provides a process of preparation of the current formulation thereof.

### BRIEF DESCRIPTION OF FIGURES:

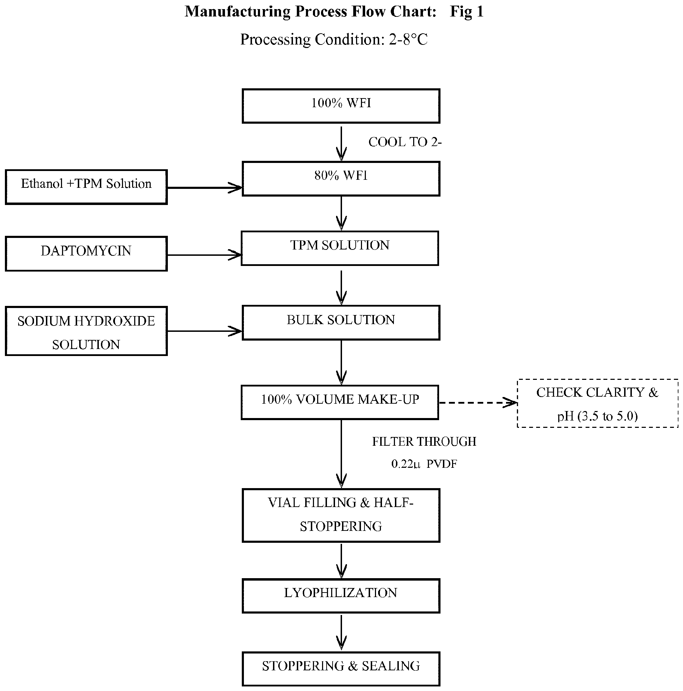

Figure 1 depicts the manufacturing process flow chart of the lyophilized daptomycin formulation of the present invention.

### DETAILED DESCRIPTION OF THE INVENTION:

The present invention is described herein after in more details substantiating various embodiments and conditions of reaction for better understanding/ appreciation of the invention.

The present invention relates to a stable, lyophilized pharmaceutical formulation comprising antibacterial agent, daptomycin as active ingredient in an amount in the range of 350mg-500 mg, tocopheryl phosphate hydrolysate mixture (TPM), wherein said lyophilized formulation is directly reconstitutable within 5 minutes for parental administration. Preferably, 0.5 % tocopheryl phosphate hydrolysate is used.

The lyophilized pharmaceutical formulation of the present invention provides an improved reconstitution time and increases stability of the reconstituted formulation at room temperature.

Tocopheryl phosphate hydrolysate mixture (TPM) used in the present formulation acts as an antioxidant as well as solubilizer which increase the stability and storage capacity of the finished product after reconstitution at room temperature for 24 hours in contrast to the innovator product which needs to be used within 12 hours of reconstitution when stored at room temperature.

Further, the present formulation can be directly reconstituted with 0.9% Sodium chloride with a reduced reconstitution time of not more than 5 minutes as compared to soaking the product for 10 minutes followed by gentle rotation or swirling the vial contents for a few more minutes for complete dissolution.

As compared with the RLD (Cubicin) marketed formulation the present invention possesses various advantages. Soak time of about 10 minutes as specified in the RLD label is not required; the product of the present invention can be directly reconstituted with a vehicle with a reduced reconstitution time of not more than 5 minutes as compared to soaking the product for 10 minutes followed by gentle rotation or swirling the vial contents for few minutes for complete dissolution.

Thus, it reduces the waiting period for the patient due to less time required for reconstitution, thereby accelerating the administration process, decreases product exposure of product to room temperature by increasing the stability, increased stability of the reconstituted injection when stored at room temperature.

The comparative reconstitution time for RLD (Cubicin) vs. In-house (IH) developed Generic 500 mg formulation (formulation composition in-line with RLD) vs. 500 mg formulation of present Invention (daptomycin with TPM) is depicted in example 3.

Further, the stability data at room temperature as well as at 2 to 8°C, of the reconstituted solution of lyophilized daptomycin injection of the present invention is illustrated in example 4. At room temperature, the reconstituted solution of the present invention was stable up to 24hours and up to 72 hours at 2 to 8°C.

The parental administration in the current invention is preferably intravenous (IV) administration.

In another embodiment, the present invention relates to a process for the preparation of lyophilized daptomycin formulation.

Accordingly, the process steps include the following;
1. 100% water for Injection was collected in a stainless steel vessel and it was cooled to between 2-8°C,
2. 80% of water for Injection from step 1 was transferred into second clean stainless steel vessel by maintaining the temperature between 2-8°C,
3. In a separate vessel TPM was solubilized in absolute ethanol (dehydrated) under continuous stirring till complete dissolution of TPM. This solution was added to water for Injection of step (2) and stirred continuously to get a milky-white solution,
4. Calculated quantity of daptomycin was added under continuous stirring to step (3) and stirred continuously till complete dissolution of daptomycin,
5. The pH of the solution was adjusted with sodium hydroxide solution if required to between 3.5 and 5.0,
6. The volume of the solution of step (5) was made up to 100% with water for injection of step (1), and
7. The above bulk solution was filtered through 0.22µ PVDF membrane filter and the filtered solution was filled into previously washed and sterilized vials, semi-stoppered with slotted lyo stoppers and was loaded into the lyophilizer.

The tocopheryl Phosphate Hydrolysate mixture (TPM) used in the present formulation comprises 0.5% tocopheryl phosphate hydrolysate, 1.7% ethanol and water quantity sufficient to 100%.

In a further embodiment, the present invention provides a method for the treatment of bacterial infections specifically Staphylococcus aureus bloodstream infections and complicated skin and skin structure infections (cSSSI) in a subject comprising administering parentally an effective amount of reconstituted lyophilized daptomycin formulation of the instant invention.

In another embodiment, the present invention relates to use of reconstituted lyophilized daptomycin formulation for the treatment of bacterial infections specifically Staphylococcus aureus bloodstream infections and complicated skin and skin structure infections (cSSSI). The subject is a human.

The following examples, which include preferred embodiments, will serve to illustrate the practice of this invention, it being understood that the particulars shown are by way of examples and for purpose of illustrative discussion of preferred embodiments of the invention only and are not limiting the scope of the invention.

### Example 1: Composition of daptomycin injection

| **Sr. No.** | **Ingredients** | **Composition per vial** |
|---|---|---|
| 1. | Daptomycin | 500 mg |
| 2. | Sodium Hydroxide | q.s to adjust pH |
| 3. | TPM ** | 2.5 mg |
| 4. | Water for Injection (WFI) | q.s 5mL |

| | | |
|---|---|---|
| **TPM composition 0.5% Tocopheryl Phosphate Hydrolysate 1.7% Ethanol Water QS 100% pH adjusted to 7 using 0.1 M NaOH. | | |

### Example 2: Process for Preparation of lyophilized daptomycin injection of the present invention

100% WFI was collected in a stainless steel vessel and cooled to between 2-8°C. 80% of WFI was maintained at temperature 2-8°C, transferred into second clean stainless steel vessel. In a separate vessel TPM was solubilized in absolute ethanol (dehydrated) under continuous stirring till complete dissolution of TPM. This solution was added to 80% of WFI and stirred continuously to get a milky-white solution. Calculated quantity of daptomycin was added to the mixture under continuous stirring till complete dissolution. The pH of the solution was adjusted with sodium hydroxide solution if required to between 3.5 and 5.0.The volume of the mixture was made up to 100% with remaining WFI. The bulk solution was filtered through 0.22µ PVDF membrane filter followed by filling the filtered solution into previously washed and sterilized vials, semi-stoppered with slotted lyo stoppers and loaded in to the lyophilizer. After lyophilization the vials were stoppered and sealed.

### Example 3: Comparative Reconstitution Studies

Given is the comparative reconstitution time for RLD (Cubicin) VS In-house (IH) developed Generic 500 mg formulation (formulation composition in-line with RLD) vs. 500 mg formulation of present Invention (daptomycin with TPM):

| **Comparative reconstitution time for RLD vs. IH developed Generic 500 mg formulation vs. 500 mg TPM formulation (formulation of present invention)** | | | | | | | |
|---|---|---|---|---|---|---|---|
| Sr. No | Strength | Formulation Type | Brand Name | Reconstitution Time (with 10 mL of 0.9% Sodium chloride injection) | | | |
| | | | | Procedure I | | | Procedure II |
| | | | | With 10 min soaking procedure | | | Direct swirling without soaking time of 10 min |
| | | | | Soaking time | Reconstitution Time | Total time | |
| 1 | 500 mg/ Vial | RLD | Cubicin | 10 min | 5 min 45 sec | 15min 45 sec | 9 min 51 sec |
| 2 | 500 mg/Vial | IH developed Generic Product | NA | 10 min | 1 min 50 sec | 11 min 50 sec | 10 min 29 sec |
| 3 | 500 mg/Vial | IH developed TPM formulation | NA | NA | NA | NA | 3 min 51sec |

### Example 4: Stability data of the reconstituted solution of lyophilized daptomycin injection of the present invention

Chemical stabilities of the reconstituted solutions of lyophilized daptomycin injection were measured by comparing measurements of assay & total impurities under known time periods & temperature conditions (e.g., up to 24 hours at room temperature and up to 72 hours at 2 to 8°C). The assay of daptomycin and total impurity for each sample was measured by high performance liquid chromatography (HPLC). In addition, the amount of daptomycin in the reconstituted daptomycin solution was measured relative to the amount of impurities selected from the group consisting of the anhydro-daptomycin the beta-isomer of daptomycin and the lactone hydrolysis product of daptomycin.

| **Period** | | **Assay by HPLC Each vial contains Daptomycin 500 mg/vial** | **Related Impurities by HPLC** | | | | |
|---|---|---|---|---|---|---|---|
| **↓** | | | **Lactone hydrolysis Product** | **Beta isomer** | **Anhydro Daptomycin** | **Any other unknown impurity** | **Total impurities** |
| **Test** | | | | | | | |
| **→** | | | | | | | |
| Initial | | 108.1% | 0.37% | 0.10% | 1.77% | 0.73% | 4.38% |
| **Room Tempe rature** | 12 Hrs | 105.8% | 0.38% | 0.10% | 2.19% | 0.72% | 4.82% |
| | 24 Hrs | 107.3% | 0.38% | 0.24% | 2.97% | 0.70% | 5.62% |
| **2°-8°C** | 48 Hrs | 104.7% | 0.35% | 0.23% | 1.26% | 0.80% | 4.27% |
| | 72 Hrs | 108.7% | 0.34% | 0.23% | 1.45% | 0.81% | 4.55% |

Unexpectedly, combining daptomycin with TPM showed enhanced chemical stability of daptomycin in reconstituted solution at different temperature conditions such as room temperature (25°C) and at 2-8°C. At room temperature, the reconstituted solution of the present invention was found to be stable for up to 24hours in contrast to the innovator product which needs to be used within 12 hours of reconstitution when stored at room temperature. Further, at 2 to 8°C the reconstituted solution of the present invention was found to be stable for up to 72 hours in contrast to the innovator product which needs to be used within 48 hours of reconstitution when stored at 2 to 8°C.

Embodiments of the invention include:
1. A stable, lyophilized formulation comprising antibacterial agent daptomycin as active and tocopheryl phosphate hydrolysate mixture (TPM), wherein, said lyophilized formulation is directly reconstitutable within 5 minutes for parental administration.
2. The stable lyophilized daptomycin formulation according to embodiment 1, wherein the daptomycin is in the range of 350-500 mg.
3. The stable lyophilized daptomycin formulation according to embodiment 1, wherein the tocopheryl phosphate hydrolysate mixture (TPM) comprises 0.5% tocopheryl phosphate hydrolysate, 1.7% ethanol, a pH adjuster 0.1 M NaOH and water.
4. A process for preparation of stable lyophilized formulation of daptomycin comprising:
   i) dissolving tocopheryl phosphate hydrolysate mixture (TPM) under continuous stirring in absolute ethanol (dehydrated) , followed by addition of this solution to water for injection at the temperature between 2-8°C;
   ii) dissolving daptomycin under continuous stirring in solution of step (i) followed by adjusting the pH to 3.5-5.0 with sodium hydroxide solution and making volume up to 100% ;
   iii) filtering solution of step (ii) and filling into sterilised vials and lyophilizing.
5. The process according to embodiment 4, wherein the tocopheryl phosphate mixture (TPM) comprises 0.5% tocopheryl phosphate hydrolysate, 1.7 % ethanol, a pH adjuster 0.1 M NaOH and water.
6. A method for the treatment of bacterial infections in a subject comprising administering parentally an effective amount of reconstituted lyophilized daptomycin formulation of embodiment 1.
7. Use of a reconstituted lyophilized daptomycin formulation of embodiment 1 in the treatment of bacterial infections in a subject.

## Claims

1. A stable, lyophilized formulation comprising antibacterial agent daptomycin as active and tocopheryl phosphate hydrolysate.

2. A formulation comprising antibacterial agent daptomycin as active and tocopheryl phosphate hydrolysate mixture (TPM).

3. A stable, lyophilized formulation obtainable by lyophilizing a composition comprising antibacterial agent daptomycin as active and tocopheryl phosphate hydrolysate mixture (TPM).

4. The daptomycin formulation according to any one of claims 1 to 3, wherein the daptomycin is in the range of 350-500 mg.

5. The daptomycin formulation according to claim 2 or 3, wherein the tocopheryl phosphate hydrolysate mixture (TPM) comprises 0.5% tocopheryl phosphate hydrolysate, 1.7% ethanol, a pH adjuster 0.1 M NaOH and water.

6. A process for preparation of stable lyophilized formulation of daptomycin comprising:
i) dissolving tocopheryl phosphate hydrolysate mixture (TPM) under continuous stirring in absolute ethanol (dehydrated), followed by addition of this solution to water for injection at the temperature between 2-8°C;
ii) dissolving daptomycin under continuous stirring in solution of step (i) followed by adjusting the pH to 3.5-5.0 with sodium hydroxide solution and making volume up to 100%;
iii) filtering solution of step (ii) and filling into sterilised vials and lyophilizing.

7. The process according to claim 6, wherein the tocopheryl phosphate mixture (TPM) comprises 0.5% tocopheryl phosphate hydrolysate, 1.7 % ethanol, a pH adjuster 0.1 M NaOH and water.

8. A stable, lyophilized formulation obtainable by the process according to claim 6.

9. A formulation according to any one of claims 1 to 5 or 8 for use in a method for the treatment of bacterial infections in a subject.
